# EUROPÄISCHE PATENTSCHRIFT

(11) **EP 1 074 228 B1**
(45) Veröffentlichungstag und Bekanntmachung des Hinweises auf die Patenterteilung: **26.04.2006**
(21) Anmeldenummer: 00116621.4
(22) Anmeldetag: 01.08.2000
(51) Int. Cl.: A61F 2/16

(54) **Einstellbare intraokulare Linse**
Adjustable intraocular lens
Lentille intraoculaire réglable

(30) Priorität: 04.08.1999 DE 19936666
(43) Veröffentlichungstag der Anmeldung: 07.02.2001
(73) Patentinhaber: Jahn, Marianne, 87435 Kempten (DE); Jahn, Claus-Ekkehard, Dr. med., 87435 Kempten (DE)
(72) Erfinder: Jahn, Marianne, 87435 Kempten (DE); Jahn, Claus-Ekkehard, Dr. med., 87435 Kempten (DE)
(74) Vertreter: Henkel, Feiler & Hänzel

(56) Entgegenhaltungen:
- EP-A- 0 162 573
- EP-A- 0 278 724
- EP-A- 0 650 704
- WO-A-93/03686
- WO-A-94/28825
- WO-A-96/15734
- DE-C- 4 116 869
- US-A- 4 787 903
- US-A- 5 288 293

## Beschreibung

### Technisches Gebiet

Die Erfindung betrifft eine intraokulare und insbesondere eine intraokulare Linse, bei der nach der Implantation der Brennpunkte nachjustiert werden kann. Die Erfindung betrifft auch ein Verfahren zur Herstellung einer derartigen Linse.

### Stand der Technik

Derzeit ist es üblich, bei Patienten, die beispielsweise an grauem Star erkrankt sind, die erkrankte und undurchsichtig gewordene Linse zu entfernen und durch ein Implantat, eine sog. intraokulare Linse (IOL, intraocular lens) zu ersetzen.

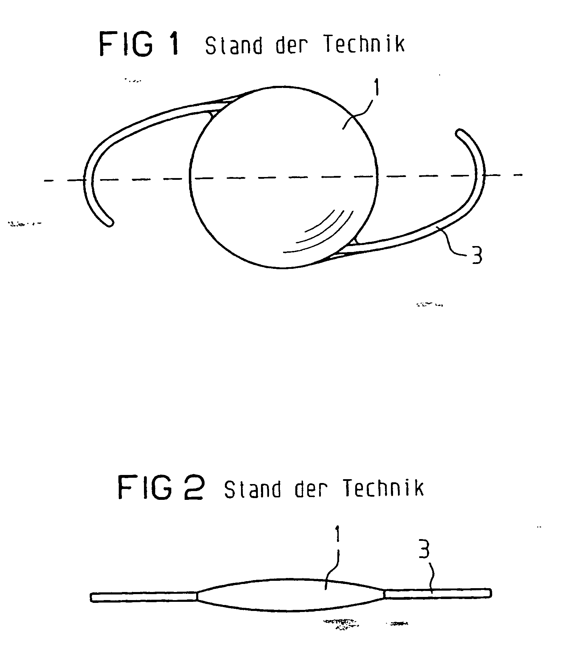

Ein Beispiel für eine derartige intraokulare Linse ist in den Fig. 1 und 2 gezeigt. Die Linse besteht aus einem Linsenkörper 1 und aus zwei mit dem Linsenkörper 1 verbundenen haptischen Armen 3. Fig. 2 zeigt eine Seitenansicht der Linse aus Fig. 1.

Vorzugsweise besteht die Linse aus einem weichen oder faltbaren Material, so daß sie entlang der gestrichelten Linie (A-A) gefaltet bzw. gerollt werden kann. Für die Implantation wird nach dem Entfernen der erkrankten Linse die intraokulare Linse in das Auge eingeführt, wobei sie bei dem Einführen entlang der gestrichelten Linie zusammengefaltet ist, so daß der Schnitt durch die Hornhaut möglichst klein ausfallen kann. Im Auge wird die Linse entfaltet und an ihrer Position, üblicherweise im Kapselsack der entfernten Linse befestigt.

Die Linsen, die üblicherweise aus einem Kunststoffmaterial, wie Polyurethanelastomer, Silikonelastomer, Hydrogelpolymer oder Collagen bestehen, können einstückig gebildet sein und sind daher preiswert herzustellen. Da sie zusammengerollt bzw. zusammengefaltet werden können, können sie durch einen kleinen Schlitz eingeführt werden, so daß das Auge nach der Operation leicht verheilt.

Ein Nachteil dieser Linsen besteht allerdings darin, daß es leicht zu Fehlanpassungen kommt, so daß der Patient auch nach der Operation eine Sehhilfe, etwa eine Brille oder Kontaktlinsen, benötigt, weil der Brennpunkt der Linse vor oder hinter die Netzhaut fällt.

Um diesem Problem zu begegnen, wurde in US 5,800,533 und in US 5,728,155 eine einstellbare intraokulare Linse vorgeschlagen. Eine solche Linse ist in Fig. 3 dargestellt. Die Linse dieses Standes der Technik, der den Oberbegriff des Anspruchs 1 bildet, besteht aus einem zylinderförmigen Linsenkörper 1 mit einem Außengewinde 5, einer Tragevorrichtung 7, die ringförmig ist und ein Innengewinde 9 aufweist, das zum Außengewinde 5 des Linsenkörpers 1 paßt, und aus haptischen Armen 3, die an der Tragevorrichtung befestigt bzw. mit dieser einstückig ausgebildet sind. Indem der Linsenkörper mehr oder weniger weit in die Tragevorrichtung eingeschraubt wird, wird der Abstand zwischen dem Linsenkörper und der Netzhaut und somit der Brennpunkt eingestellt.

US 5,728,155 zeigt auch, daß ein flexibler Linsenkörper in die Tragevorrichtung eingesetzt werden kann, so daß ein späterer Austausch der Linse mit einem kleinen Schnitt durch die Hornhaut möglich ist. Auch offenbart diese Druckschrift, daß der Linsenkörper relativ zur Tragevorrichtung auch mit anderen Mitteln als einem Innen- und Außengewinde verstellt werden kann. Beispielsweise kann die Linse mit einem an der Tragevorrichtung befestigten Schneckengetriebe bewegt werden.

Diese genannten einstellbaren intraokularen Linsen haben jedoch eine Reihe von Nachteilen. Zum einen sind sie schwierig herzustellen, da sie aus mehreren Teilen bestehen, die mit sehr geringen Toleranzen zueinander passen müssen. Insbesondere sind Verschiebemechanismen mittels Schneckengetrieben und ähnlichem aufwendig, kompliziert und teuer in der Herstellung.

Ein weiterer Nachteil besteht darin, daß der Linsenkörper in einer ringförmigen Tragevorrichtung gehalten ist. Diese ist für gewöhnlich starr, so daß bei der Implantation ein großer Schnitt nötig ist. Auch reicht die Tragevorrichtung nah an das Blickfeld des Patienten heran, so daß durch die dicke Tragevorrichtung unerwünschte Reflexe und Beeinträchtigungen auftreten können.

Weitere ferstellbare intraokulare Linsen sind aus US 5,203,788 und aus US 5,288,293 bekannt. Insbesondere zeigt US 5,288,293 eine intraokulare Linse, bei der durch ein Einstellmittel der Winkel zwischen den haptischen Armen und dem Linsenkörper einstellbar ist, so daß der Linsenkörper relativ zu den haptischen Armen in Richtung der optischen Achse bewegbar ist. Das Einstellmittel ist ein durch Laserlicht im Volumen veränderbares Material, und die Einstellung erfolgt durch Einstrahlen von Laserlicht. Diese Art der Einstellung ist relativ ungenau und schwierig, da das Einstellmittel nur einmal verstellt werden kann. Außerdem kann das am Rand des Linsenkörpers angebrachte große Einstellmittel zu störenden Reflexen führen.

WO 93 03686 zeigt eine intraokulare Linse gemäß dem Oberbegriff von Anspruch 1. Diese Linse weist einen Führungsstift auf, der in eine entsprechende Ausnehmung eingreift, um als Führungselement bei einer Verstellung der Linse zu dienen. Die eigentliche Verstellung erfolgt über einen Stellschraubenmechanismus.

US 5 288 293 zeigt eine weitere verstellbare intraokulare Linse, bei der die Verstellung mittels eines Hydrogel- oder Kollagenmaterialklumpens erzielt wird, der an der Verbindungsstelle zwischen haptischem Arm und Linse angebracht ist. Durch Einstrahlen von Laserlicht wird der Winkel zwischen dem haptischen Arm und der Linse, etwa durch verdampfen des Hydrogels, verändert.

WO 96 15734 zeigt eine weitere verstellbare intraokulare Linse, bei der am Rand des Linsenkörpers ein Durchgangsloch ausgebildet ist, in dem das Ende eines haptischen Arms aufgenommen ist. Die Verstellung der Linse erfolgt jedoch durch Verformung der haptischen Arme selbst.

Der Erfindung liegt die Aufgabe zugrunde, eine einstellbare intraokulare Linse zu schaffen, die einfach und preiswert herzustellen ist und die genannten Nachteile vermeidet.

### Zusammenfassung der Erfindung

Die Aufgabe wird durch eine einstellbare intraokulare Linse nach Anspruch 1 oder 5 gelöst. Die abhängigen Ansprüche betreffen weitere vorteilhaft Ausgestaltungen der Erfindung.

Entsprechend einem weiteren vorteilhaften Aspekt der Erfindung kann die gesamte Linse faltbar bzw. aufrollbar sein, so daß sie durch einen kleinen Schnitt in der Hornhaut eingesetzt werden kann.

Ein weiterer Aspekt der Erfindung sieht vor, die Einstellung der Linse mittels eines elektrischen Motors, etwa eines piezoelektrischen Elementes, durchzuführen.

Ein Vorteil der Erfindung liegt in der Bereitstellung einer einstellbaren intraokularen Linse mit einem Verstellmechanismus, der optisch nicht stört.

### Kurze Beschreibung der Zeichnungen

Die Erfindung wird nun anhand der beiliegenden Zeichnungen erläutert, in denen zeigt:
- Fig. 1: eine Aufsicht auf eine nicht einstellbare bekannte Linse;
- Fig. 2: eine Seitenansicht der Linse aus Fig. 1;

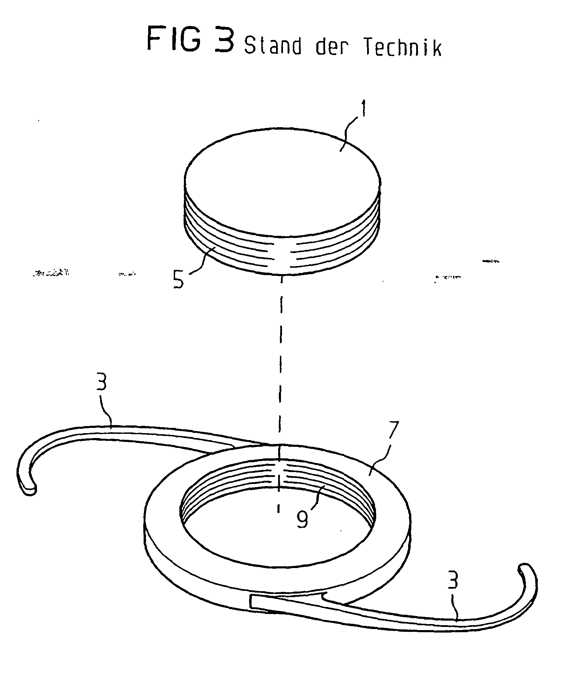

- Fig. 3: eine einstellbare Linse entsprechend dem Stand der Technik;
- Fig. 4: eine Querschnittsansicht durch ein menschliches Auge, in dem der Linsenkörper entfernt wurde;
- Fig. 5: die Aufsicht auf eine erfindungsgemäße Linse;
- Fig. 6a: eine Seitenansicht einer Linse mit bei maximaler Verstellung, die nicht Gegenstand dieser Anmeldung ist.
- Fig. 6b: eine Seitenansicht dieser Linse mit bei minimaler Verstellung
- Fig. 7: die Seitenansicht einer weiteren Linse, die nicht Gegenstand dieser Anmeldung ist;
- Fig. 8: die Seitenansicht einer erfindungsgemäßen Linse;
- Fig. 9: die Seitenansicht einer Linse, die nicht Gegenstand dieser Anmeldung ist;
- Fig. 10: die Seitenansicht einer weiteren Linse, die nicht Gegenstand dieser Anmeldung ist,
- Fig. 11: die Seitenansicht einer weiteren Linse, die nicht Gegenstand dieser Anmeldung ist;
- Fig. 12: die Seitenansicht einer weiteren Linse, die nicht Gegenstand dieser Anmeldung ist;
- Fig. 13: die Seitenansicht der erfindungsgemäßen Linse;
- Fig. 14: eine Ansicht eines modifiriesten Einstellmittels;
- Fig. 15: die Seitenansicht einer weiteren Linse, die nicht Gegenstand dieser Anmeldung ist;
- Fig. 16: die Seitenansicht einer weiteren Linse, die nicht Gegenstand dieser Anmeldung ist;
- Fig. 17: eine Aufsicht auf eines der Gabelenden aus Fig. 16;
- Fig. 18: eine Seitenansicht einer weiteren Linse, die nicht Gegenstand dieser Anmeldung ist; und
- Fig. 19: eine Ansicht einer weiteren erfindungsgemäßen Linse.

### Ausführliche Beschreibung der Erfindung

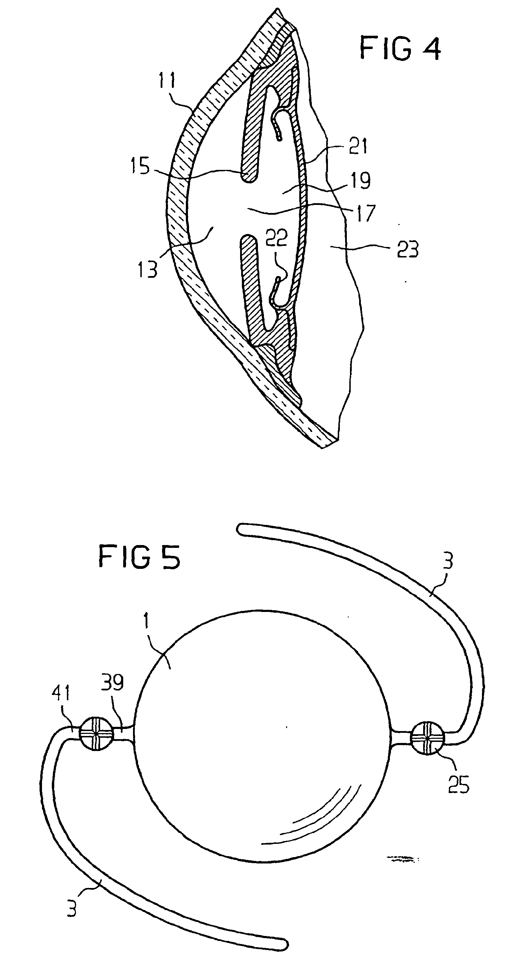

In Fig. 4 ist das menschliche Auge im Schnitt dargestellt, wobei der Linsenkörper entfernt wurde. Hinter einer aus klarem Gewebe gebildeten Hornhaut 11 befindet sich die vordere Augenkammer 13. Diese wird von der Iris 15 und der Pupille 17 begrenzt. Daran schließt sich die hintere Augenkammer 19 an, die ihrerseits auf der einen Seite von der Iris 15 und der Pupille 17 und andererseits von der Glashaut 21 und dem Glaskörper 23 begrenzt wird. Üblicherweise wird die intraokulare Linse in der hinteren Augenkammer angebracht, wobei die haptischen Arme 3 sich gegen den Rand der hinteren Augenkammer oder gegen den Kapselsack 22 abstützen und die Linse in Position hält.

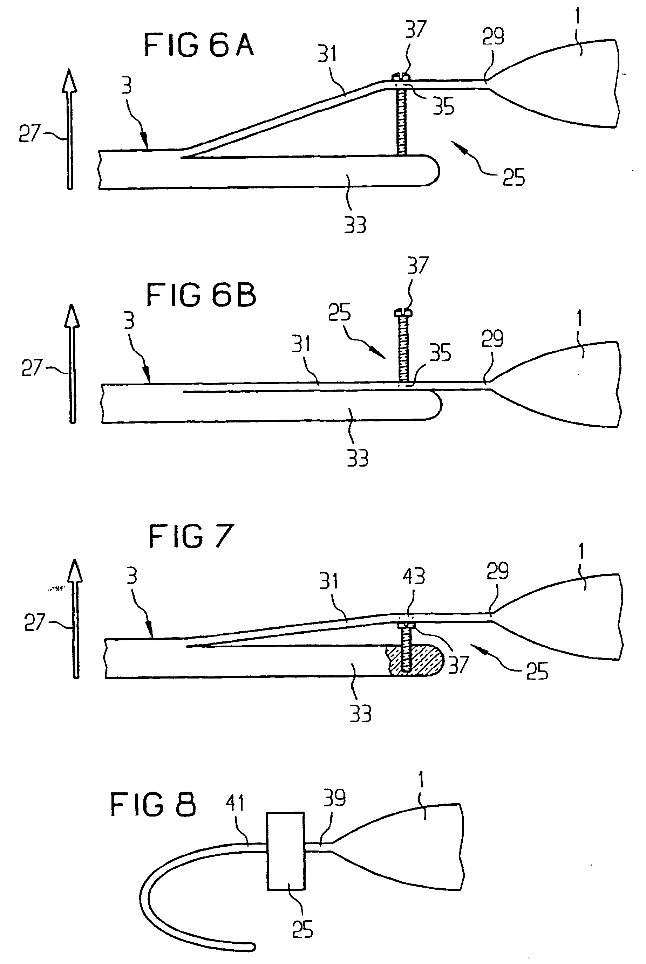

Fig. 5 zeigt eine erfindungsgemäße Linse in der Aufsicht. Die Linse besteht aus einem Linsenkörper 1 an den sich zwei einander gegenüberliegende haptische Arme 3 anschließen. Die haptischen Arme 3 weisen Einstellmittel 25 auf, um den Linsenkörper 1 gegenüber den haptischen Armen 3 in Richtung der optische Achse 27 des Linsenkörpers 1 verschiebbar zu halten und das Ausmaß der Verschiebung einzustellen.

Fig. 6a zeigt eine Seitenansicht eines Teils einer Linse, die nicht Gegenstand dieser Anmeldung ist, wobei einer der haptischen Arme 3 mit dem Einstellmittel 25 in der maximal ausgefahrenen Position dargestellt ist, während Fig. 6b den selben Teil der Linse mit dem haptische Arm 3 und dem Einstellmittel 25 in der maximal eingefahrenen Position zeigt.

Der haptische Arm 3 weist an seinem dem Linsenkörper zugewandten Ende 29 eine Aufgabelung in zwei Gabelenden 31, 33 auf. Das Gabelende 31 ist mit dem Linsenkörper 1 verbunden. Es ist zumindest an seinen Verbindungsstellen mit dem Linsenkörper 1 und dem anderen Gebelende 33 derart flexibel ausgestaltet, daß eine Relativbewegung der beiden Gabelenden zueinander möglich ist. Vorzugsweise ist der gesamte haptische Arm 3 aus einem Kunststoffmaterial mit einer gewissen Elastizität gebildet. Des weiteren ist vorzugsweise das andere Gabelende 33 steifer als das Gabelende 21. Dies kann durch eine entsprechende Materialwahl oder durch entsprechende Dicken der beiden Gabelenden verwirklicht sein. In der gezeigten Ausführungsform ist das Gabelende 33 um ein Vielfaches dicker als das Gabelende 31.

Das Gabelende 33 ist an seinem der Linse zugewandten Ende mit einer agberundeten Kante versehen, so daß beim Einsetzen der Linse kein Vorsprung vorhanden ist, an dem sich die Linse mit der Iris oder der Hornhaut verharken könnte.

Der hintere Teil der haptischen Arme 3, von dem sich die beiden Gabelenden 31, 33 erstrecken, kann aus einem relativ weichen, elastichen Material gebildet sein. Auch der Einsatz eines Materials mit "Memory"-Effekt, das nach einer Verformung bei Erwärmung wieder in die Ursprungsform zurückkehrt, ist möglich.

Das Gabelende 31 trägt bei dieser Ausführungsform das Einstellmittel 25. Dieses besteht aus einem Gewindeloch 35 in Richtung der optischen Achse 27 durch das Gabelende 31. Eine Einstellschraube 37 ist in das Gewindeloch 35 derart eingeschraubt, daß ihr vorderes Ende am anderen Gabelende 33 anliegt und durch die Federkraft der elastischen Gabelelemente 31, 33 angepreßt wird.

Wie aus Fig. 6a und Fig, 6b zu sehen ist, ändert sich die Relativposition des Linsenkörpers 1 gegenüber dem Gabelende 33 mit dem Herein- bzw. Herausschrauben der Einstellschraube 37. Dementsprechend kann der Augenchirurg nach Einsetzen der einstellbaren intraokularen Linse den Brennpunkt der Linse genau auf die Netzhaut des Patienten einstellen.

Hierfür kann zunächst die intraokulare Linse ohne Einstellschraube, das heißt mit aneinander liegenden Gabelenden 31, 33, in das Auge eingesetzt werden. Nachdem die Folgen dieser Operation verheilt sind, kann die Fehlsichtigkeit des Patienten bestimmt. Nun kann in einem kleinen Eingriff mit örtlicher Betäubung die Hornhaut unmittelbar über dem Gewindeloch 35 geöffnet werden und Einstellschrauben 37 mit der erforderlichen Länge eingeschraubt werden. Hierfür ist nur ein sehr kleiner Schnitt durch die Hornhaut nötig, der außerhalb des Blickfelds liegt und schnell verheilt.

Alternativ dazu kann die intraokulare Linse mit der eingesetzten Einstellschraube 37 eingesetzt werden, wobei vorzugsweise die Einstellschraube bis etwa zur Hälfte eingeschraubt ist.

Durch Auswahl einer Schraube der geeigneten Länge kann problemlos eine Einstellung der Linse um bis zu 5 Dioptren (3,25 mm) erzielt werden.

Die Linse kann in einem Gießschritt hergestellt werden, wobei anschließend durch einen Schnitt durch die haptischen Arme 3 die Aufgabelung in die Gabelenden 31, 33 ausgebildet wird. Anschließend wird ein Gewindeloch 35 in die Aufgabelung 31 eingeschnitten. Alternativ dazu kann bereits bei einem Gießschritt die Linse mit samt ihrer haptischen Arme 3, der Aufgabelung und dem Gewindeloch 35 ausgebildet werden.

Die erfindungsgemäße intraokulare Linse ist preiswert herzustellen, unkompliziert und einfach zu handhaben und bietet eine große Einstellmöglichkeit für den Chirurgen.

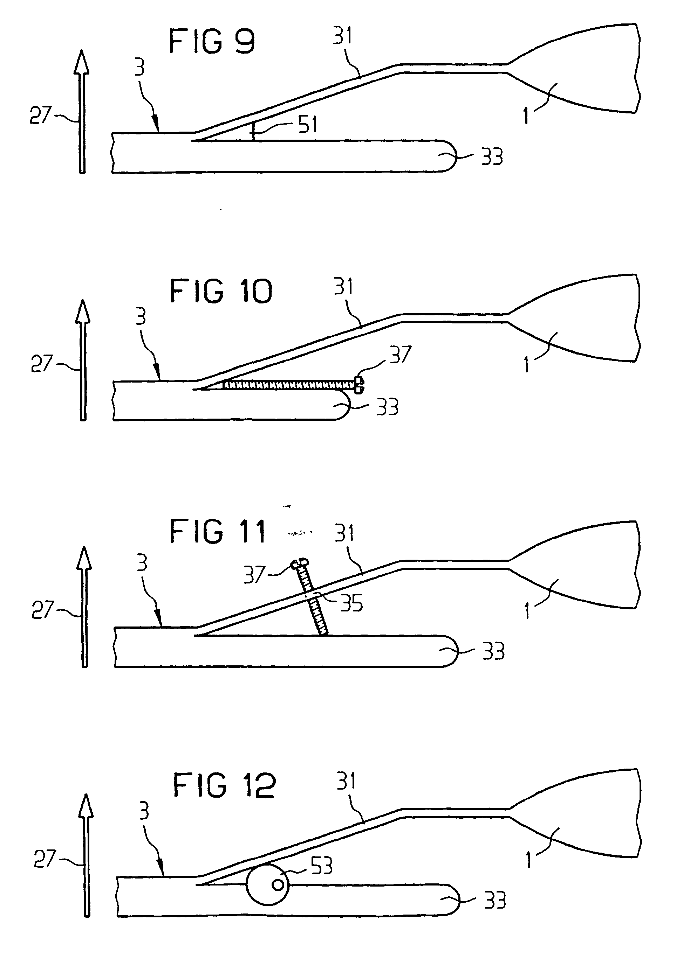

Fig. 7 zeigt eine weitere Linse. Die in Fig. 8 gezeigte Linse unterscheidet sich von derjenigen aus den Fig. 6a und 6b in den folgenden Punkten. Das Einstellmittel dieser Ausführungsform besteht aus einem gewindefreien Durchgangsloch 43 in dem Gabelende 31, einer Einstellschraube 37 und einem nicht-durchgehenden Gewindeloch 45 an dem anderen Gabelende 33. Die Verstellung des Linsenkörpers 1 erfolgt hier, indem durch das gewindefreie Durchgangsloch 43 ein Werkzeug in den Kopf der Einstellschraube 37 eingreift, der von unten an dem Gabelende 31 - also im Zwischenraum zwischen den beiden Gabelenden 31, 33 - anliegt. Durch Drehen der Schraube 25 kann das Gabelende 31 gegenüber dem Gabelende 33 gehoben bzw. abgesenkt werden. Da bei dem haptischen Arm 3 das Gabelende 33 mit wesentlich größerer Dicke als das Gabelende 31 ausgebildet ist, ist auf diese Art eine Verstellung über einen großen Bereich möglich, ohne die Schraube auszuwechseln und ohne störende Vorsprünge, die von der Linse hervorspringen und möglicherweise das Auge verletzen.

Neben den gezeigten Linsen bieten sich weitere Verstellmöglichkeiten an. Beispielsweise kann die Schraube in den Linsen der Fig. 6a, 6b und 8 durch ein Piezoelement ersetzt werden, das die beiden Gabelenden 31, 37 voneinander weg drückt. Alternativ dazu können Abstandselemente, beispielsweise in Keilform, zwischen die beiden Gabelenden geschoben werden. Auch ist es möglich, ein elastisches fluidgefülltes Kissen zwischen die beiden Gabelenden zu positionieren und für die Einstellung Fluid zuzuführen oder abzusaugen.

Auch können die haptischen Arme 3 aus zwei Armschichten bestehen, die über einen ersten Abschnitt miteinander fest verbunden sind.

Auch kann der weiche Linsenkörper 1 einen festen dünnen Außenring aufweisen, der vorzugsweise über zwei Scharniere zusammen mit der Linse zusammengefaltet werden kann. Dieser Außenring kann außerdem zwei Riegelelemente tragen, die nach der Implantation den Linsenkörper in der entfalteten Stellung halten.

Fig. 8 zeigt die Seitenansicht einer erfindungsgemäßen Linse. Bei der gezeigten Linse sind die haptischen Arme 3 nicht aufgegabelt, sondern weisen ein Einstellmittel 25 auf. Die haptischen Arme 3 sind zweigeteilt. Ein erster Abschnitt 39 erstreckt sich zwischen dem Linsenkörper 1 und dem Einstellmittel 25. Ein zweiter Abschnitt 41 erstreckt sich vom Einstellmittel 25 von der Linse weg.

Weitere unterschiedliche Ausgestaltungen einer intraokularen Linse sind in den Fig. 9 bis 19 gezeigt.

Die einstellbare intraokulare Linse aus Fig. 9 weist ein keilförmiges Abstandselement 53 zwischen den Gabelenden 31, 33 auf, das durch Verschieben die Linsenposition ändern kann.

Weitere einstellbare intraokulare Linsen sind in den Fig. 10 und 11 gezeigt. Hierbei ist die Einstellschraube 37, das Gewindeloch 35 und das Durchgangsloch 43 in bezug zur optischen Achse 27 geneigt. Dadurch verschiebt sich der Eingriffspunkt auf der Hornhaut für eine postoperative Justierung nach außen und weg vom Sichtfeld des Patienten.

Bei der Linse nach Fig. 12 ist eine Exzenterscheibe als Abstanselement 53 vorgesehen.

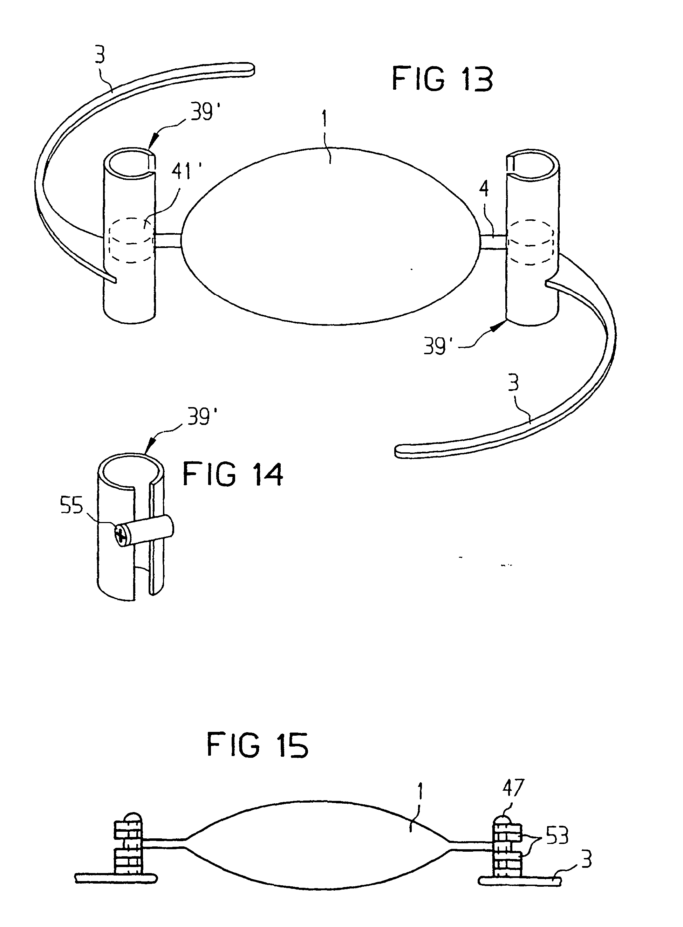

Bei der erfindungsgemäßen Linse nach Fig. 13 hat das Einstellmittel die Form einer Klemmführung parallel zur optischen Achse 27.

Bei dieser bevorzugten Ausführungsform weist der eine Abschnitt 41 des haptischen Arms, der mit einem seiner Enden an der Linse angebracht ist, an dem anderen Ende ein zylinder- oder scheibenförmiges Endstück 41' auf, das mit der optischen Achse ausgerichtet ist. Der andere Abschnitt 39 des haptischen Arms weist an seinem einen Ende ein hohlzylinderförmiges bzw. flanschartiges Endstück 39' auf, das das scheibenförmige Endstück 41' umschließt. Durch einen vorzugsweise durchgehenden Schlitz in der Wand des Endstücks 39' ragt der Abschnitt 41 des haptischen Arms hindurch.

Der Innenraum des hohlzylinderförmigen Endstücks 39' und die Außenkontur des scheibenförmigen Endstücks 41' sind aufeinander abgestimmt. Durch Klemmwirkung kann das Endstück 41' im anderen Endstück 39' gehalten werden, wobei die relative Lage zueinander mechanisch verstellbar ist.

Zusammen bilden die beiden Endstücke 39' und 41' das Einstellmittel. Vorzugsweise sind die beiden Endstücke 39' und 41' derart ausgeformt, daß sie gegeneinander nicht verdrehbar sind, beispielsweise können der Hohlzylinder und die Scheibe jeweils ovale Formen haben. Jedoch sind auch andere Formen möglich, etwa Halbkrise, Vielecke oder "Hundeknochen". In dieser Beschreibung ist der Begriff "Hohlzylinder" deshalb nicht einschränkend als Kreiszylinder zu verstehen. Auch kann das Prinzip der Klemmführung so abgewandelt werden, daß ein relativer dünner Flansch als ein Endstück einen längeren Stift als anderes Endstück umfaßt.

Das hohlzylinderförmige Endstück 39' und der Abschnitt 41 des haptischen Arms 3 kann aus Metall einstückig gefertigt werden. Während das scheibenförmige Endstück 41' zusammen mit der Linse - vorzugsweise einstückig - aus einem Kunststoff gefertigt ist.

Weiterhin ist es zu bevorzugen, den Abschnitt 39 und das Endstück 39' mit einem biologisch verträglichen Kunststoff zu ummanteln.

Die Ausführung des hohlzylinderförmigen Endstücks 39' aus Metall mit einem durchgehenden Längsschlitz in der Außenwand hat den Vorteil einer elastischen bzw. federnden Anpassung des Endstücks 39' an das darin aufgenommenen Endstück 41'.

Alternativ dazu kann das hohlzylinderförmige Endstück auch an dem Abschnitt 41 des haptischen Arms, also auf der Seite des Linsenkörpers ausgebildet sein.

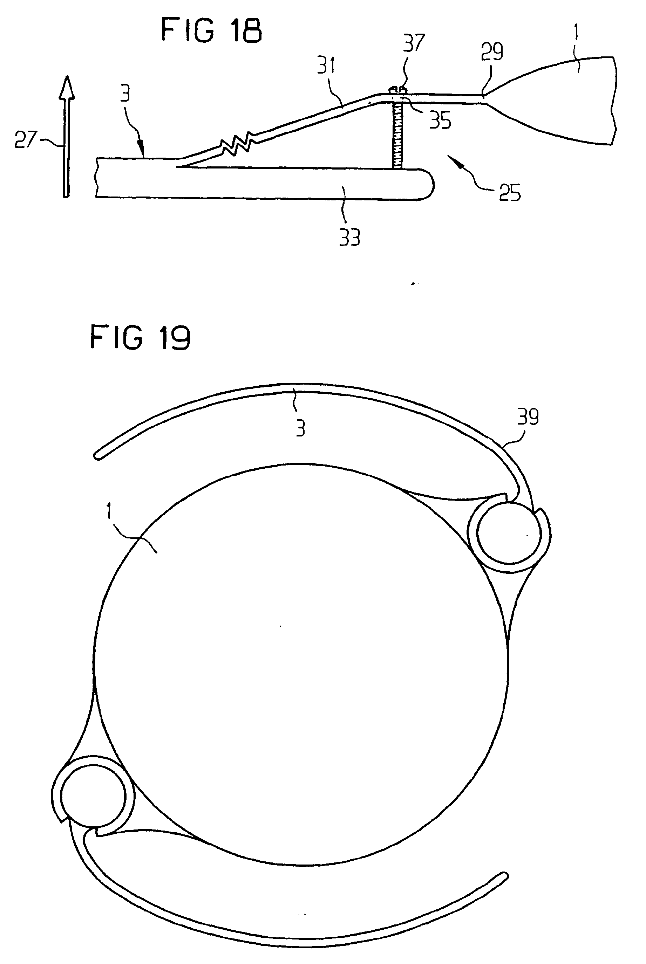

In einer in Fig. 19 gezeigten, derzeit bevorzugten Ausführungsform wird dazu ein Durchgangsloch im Rand des Linsenkörpers, parallel zur optischen Achse, ausgebildet. Ein Längsschlitz ermöglicht die Verbindung mit dem anderen Abschnitt 39 des haptischen Arms. Anders gesagt, bei dieser Ausführungsform ist das Einstellmittel im Rand des Linsenkörpers ausgebildet, und nicht über einen Abschnitt 41 des haptischen Arms mit dem Linsenkörper verbunden.

Fig. 14 zeigt als weitere Möglichkeit, daß nach dem Positionieren die Linse durch ein Arretiermittel 55, etwa eine Schraube, festgelegt werden kann.

Entsprechend der einstellbare intraokulare Linse nach Fig. 15 hat das Einstellmittel 25 einen in Richtung der optischen Achse 27 verlaufenden Stift 47, der mit einem der zwei Abschnitte 39 der haptischen Arme 3 festverbunden ist, während der andere Abschnitt 41 entlang des Stifts 47 verschiebbar gelagert ist.

Hufeisenförmige Abstandscheiben 53 können zwischen den beiden Abschnitten 39, 41 klemmend auf den Stift 47 aufgeschoben werden und den Abstand zwischen den Abschnitten 39, 41 festlegen.

Alternativ dazu können, wie erwähnt, die Abschnitte 39, 41 auch durch Klemmwirkung in ihrer relativen Position gehalten werden.

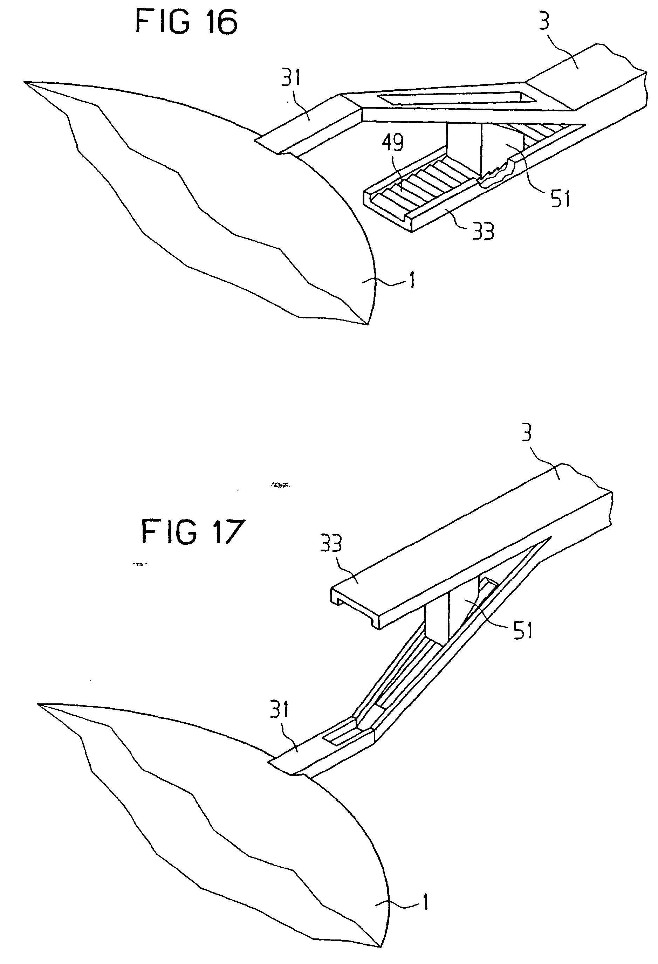

Entsprechend der intraokulare Linse nach Fig. 16 hat mindestens eines der Gabelenden 31, 33 an der dem anderen Gabelende 33, 31 zugewandten Seite eine Zahnung 49 - ähnlich einer Zahnstange, wobei ein Abstandselement 51 entlang der Gabelenden 31, 33 bewegbar ist und in seiner Position durch Eingriff in die Zahnung festgelegt ist. Fig. 17 zeigt eine Aufsicht auf das nach außen gerichtete Gabelende (31). Dieses ist vorzugsweise mit einem mittleren Längsschlitz ausgestattet, durch den hindurch der Zugriff auf das Abstandselement erfolgen kann.

In Fig. 18 ist eine weitere Linse gezeigt. Diese unterscheidet sich von der Linse aus Fig. 6a und 6b dadurch, daß in dem Gabelende 31 zwischen dem Gewindeloch 35 und der Aufgabelungsstelle ein Dehnungselement 55 vorgesehen ist, um leichter die Längenänderung des Gabelendes 31 aufnehmen zu können. Ein solches Dehnungselement kann auch bei den anderen Ausführungsformen der Erfindung an den passenden Stellen vorgesehen werden.

In Fig. 18 ist das Dehnungselement ein ziehharmonikaförmiger Abschnitt im Gabelende 31.

Obwohl in der vorangehenden Beschreibung einige bevorzugte Ausführungsformen dargestellt sind, ist die Erfindung nicht auf diese beschränkt. Änderungen und Abwandlung im Rahmen der fachmännischen Wissens sind mit umfaßt.

## Patentansprüche

1. Einstellbare intraokulare Linse mit:
einem Linsenkörper (1);
haptischen Armen (3), um den Linsenkörper (1) im Auge festzulegen; und
einem Einstellmittel (25), um den Linsenkörper (1) in Richtung der optischen Achse (27) des Linsenkörpers (1) mehrfach und reversibel verstellbar zu halten;
wobei die haptischen Arme (3) jeweils an einem Ende mit dem Linsenköprper verbunden sind, und
wobei die haptischen Arme (3) jeweils in zwei Anschnitte (39, 41) unterteilt sind, die über das Einstellmittel (25) verbunden sind, und wobei die haptischen Arme (39, 41) die Endstücke (39', 41') aufweisen;
wobei das Einstellmittel (25) ein zylinder- oder scheibenförmiges Endstück (41') und ein hohlzylinder- bzw. flanschartiges Endstück (39') aufweist, das das zylinder- oder scheibenförmige Endstück (41') umschließt, wobei die Endstücke (39', 41') zusammen das Einstellmittel (25) bilden; und
**dadurch gekennzeichnet, daß**
das eine Endstück (41') durch Klemmwirkung im anderen Endstück (39') gehalten wird, und ihre relative Lage zueinander mechanisch verstellbar ist.

2. Einstellbare intraokulare Linse nach Anspruch 1, **dadurch gekennzeichnet, daß** der Linsenkörper faltbar oder rollbar ist.

3. Einstellbare intraokulare Linse nach Anspruch 1, **dadurch gekennzeichnet, daß** das Einstellmittel (25) ein Piezoelement aufweist, das die Verstellung des Einstellmittels (25) bewirkt.

4. Einstellbare intraokulare Linse nach Anspruch 1, **dadurch gekennzeichnet, daß** mindestens einer der haptischen Arme (3) ein Dehnungselement (55) aufweist.

5. Einstellbare intraokulare Linse mit:
einem Linsenkörper (1);
haptischen Armen (3), um den Linsenkörper (1) im Auge festzulegen; und
einem Einstellmittel (25), um den Linsenkörper (1) in Richtung der optischen Achse (27) des Linsenkörpers (1) mehrfach und reversibel verstellbar zu halten;
wobei ein Teil des Einstellmittels (25) in Form eines Durchgangslochs am Rande des Linsenkörpers (1) ausgebildet ist, in welches ein Endstück (41') eines haptischen Arms (3) axial eingreift,;
**dadurch gekennzeichnet, daß**
das Endstück (41') zylinder- oder scheibenförmig ist und durch Klemmwirkung im Durchgangsloch gehalten wird, wobei die relative Lage zueinander mechanisch verstellbar ist.

## Claims

1. An adjustable intraocular lens comprising:
a lens body (1);
haptic arms (3), to fix the lens body (1) in the eye; and
an adjustment means (25) to hold the lens body (1) so as to be repeatedly and reversibly displaceable in the direction of the optic axis (27) of the lens body (1);
the haptic arms (3) each being connected at one end to the lens body, and
the haptic arms (3) each being subdivided into two portions (39, 41) connected via the adjustment means (25), and the haptic arms (39, 41) comprising the end pieces (39', 41');
the adjustment means (25) having a cylindrical or disc-shaped end piece (41') and a hollow-cylindrical or flange-like end piece (39') which encloses the cylindrical or disc-shaped end piece (41 '), the end pieces (39', 41') together forming the adjustment means (25);
and
**characterised in that**
one end piece (41') is held by a clamping effect in the other end piece (39'), and their relative position to one another is mechanically displaceable.

2. An adjustable intraocular lens according to Claim 1, **characterised in that** the lens body is foldable or rollable.

3. An adjustable intraocular lens according to Claim 1, **characterised in that** the adjustment means (25) has a piezoelectric element which effects the displacement of the adjustment means (25).

4. An adjustable intraocular lens according to Claim 1, **characterised in that** at least one of the haptic arms (3) has an expansion element (55).

5. An adjustable intraocular lens comprising:
a lens body (1);
haptic arms (3) to fix the lens body (1) in the eye; and
an adjustment means (25) to hold the lens body (1) so as to be repeatedly and reversibly displaceable in the direction of the optic axis (25) of the lens body (1);
a part of the adjustment means (25) being designed in the form of a through-hole at the edge of the lens body (1), into which an end piece (41') of a haptic arm (3) engages axially;
**characterised in that**
the end piece (41') is cylindrical or disc-shaped and is held in the through-hole by a clamping effect, the relative position to one another being mechanically displaceable.

## Revendications

1. Lentille intraoculaire réglable comprenant :
- un corps de lentille (1) ;
- des bras haptiques (3) destinés à fixer le corps de lentille (1) dans l'oeil ; et
- un moyen de réglage (25) destiné à maintenir le corps de lentille (1) dans une position réglable à plusieurs reprises et de façon réversible dans la direction de l'axe optique (27) du corps de lentille (1) ;
où les bras haptiques (3) sont chacun reliés à une extrémité avec le corps de lentille, et
où les bras haptiques (3) sont chacun divisés en deux sections (39, 41), qui sont reliées par le biais du moyen de réglage (25), et où les bras haptiques (39, 41) présentent les pièces d'extrémité (39', 41') ;
où le moyen de réglage (25) présente une pièce d'extrémité en forme de cylindre ou de disque (41') et une pièce d'extrémité semblable à un cylindre creux ou à une collerette (39') qui entoure la pièce d'extrémité en forme de cylindre ou de disque (41'), les pièces d'extrémité (39', 41') formant ensemble le moyen de réglage (25) ;
**caractérisée en ce qu'**une pièce d'extrémité (41') est maintenue dans l'autre pièce d'extrémité (39') par effet de serrage et **en ce que** leur position relative l'une par rapport à l'autre est réglable mécaniquement.

2. Lentille intraoculaire réglable selon la revendication 1, **caractérisée en ce que** le corps de lentille est pliable ou enroulable.

3. Lentille intraoculaire réglable selon la revendication 1, **caractérisée en ce que** le moyen de réglage (25) présente un piézoélément qui effectue l'ajustement du moyen de réglage (25).

4. Lentille intraoculaire réglable selon la revendication 1, **caractérisée en ce qu'**au moins l'un des bras haptiques (3) présente un élément d'extension (55).

5. Lentille intraoculaire réglable ayant :
- un corps de lentille (1) ;
- des bras haptiques (3) destinés à fixer le corps de lentille (1) dans l'oeil ; et
- un moyen de réglage (25) destiné à maintenir le corps de lentille (1) dans une position réglable à plusieurs reprises et de façon réversible dans la direction de l'axe optique (27) du corps de lentille (1) ;
où une partie du moyen de réglage (25) est réalisée sous la forme d'un trou de passage sur le bord du corps de lentille (1), dans lequel une pièce d'extrémité (41') d'un bras haptique (3) s'engage axialement ;
**caractérisée en ce que** la pièce d'extrémité (41') a une forme de cylindre ou de disque et **en ce qu'**elle est maintenue dans le trou de passage par effet de serrage, la position relative l'une par rapport à l'autre étant réglable mécaniquement.
